# FASCICULE DE BREVET EUROPEEN

(11) **EP 1 068 851 B1**
(45) Date de publication et mention de la délivrance du brevet: **15.09.2004**
(21) Numéro de dépôt: 00401574.9
(22) Date de dépôt: 05.06.2000
(51) Int. Cl.: A61K 7/00, B01F 17/00

(54) **Composition sous forme d'émulsion eau-dans-huile et ses utilisations cosmétiques**
Zusammensetzung vom Typ Wasser-in-Öl Emulsion und ihre kosmetische Verwendungen
Emulsion composition of the type water-in-oil and its cosmetic uses

(30) Priorité: 12.07.1999 FR 9909012
(43) Date de publication de la demande: 17.01.2001
(73) Titulaire: L'OREAL, 75008 Paris (FR)
(72) Inventeur: Afriat, Isabelle, 75003 Paris (FR); Boulier, Virginie, 95520 Osny (FR)
(74) Mandataire: Rasson, Catherine

(56) Documents cités:
- EP-A- 0 612 517
- EP-A- 0 670 157
- EP-A- 0 965 331
- EP-A- 0 970 682
- WO-A-93/14742
- WO-A-95/15812
- WO-A-99/47111

## Description

L'invention se rapporte à une composition se présentant sous forme d'une émulsion eau-dans-huile (E/H) comportant une forte teneur en eau et un tensioactif siliconé particulier. Cette composition a l'aspect d'une crème et est utilisable en particulier dans les domaines cosmétique et/ou dermatologique.

Dans les domaines cosmétique ou dermatologique, il est courant d'utiliser des compositions ayant l'aspect d'une crème et constituées d'une émulsion eau-dans-huile (E/H) comportant une phase aqueuse dispersée dans une phase huileuse. Une crème est, dans les domaines considérés, une composition présentant une certaine viscosité, par opposition aux compositions liquides ou semi-liquides telles que les lotions et les laits, ou encore aux compositions solides.

Les émulsions E/H comportent une phase continue huileuse et permettent donc de former à la surface de la peau un film lipidique qui prévient la perte d'eau transépidermique et protège la peau des agressions extérieures. Ces émulsions sont particulièrement appropriées pour protéger et nourrir la peau, et en particulier pour traiter les peaux sèches.

Toutefois, les crèmes sous forme d'émulsions E/H présentent l'inconvénient d'être inconfortables du fait de la sensation grasse et lourde apportée par cette phase grasse externe qui subsiste sur la peau. Ainsi, ces crèmes sont en général utilisées pour les peaux sèches, étant trop grasses pour être utilisées sur les peaux grasses. De plus, elles n'apportent pas de fraîcheur et sont généralement trop riches en huiles pour être utilisées pendant l'été ou dans les pays chauds.

Pour surmonter ces inconvénients, il a été envisagé de préparer des émulsions E/H à forte teneur en eau. Toutefois, la teneur en eau ne peut pas être trop importante pour des raisons de stabilité, ou alors une forte teneur en eau doit être compensée par l'ajout de plusieurs tensioactifs ou d'agents gélifiants qui peuvent nuire au confort de la composition finale et même entraîner des problèmes d'irritations cutanés notamment chez les sujets à peaux sensibles.

Il subsiste donc le besoin d'une composition ayant la viscosité d'une crème et se présentant sous forme d'une émulsion eau-dans-huile stable, comportant une quantité importante d'eau et utilisable dans les domaines cosmétique et/ou dermatologique, qui ne présente pas les inconvénients de l'art antérieur.

La demanderesse a maintenant trouvé de manière surprenante que l'on pouvait obtenir une composition du type émulsion eau dans huile permettant d'atteindre ces objectifs, et notamment contenant une grande quantité d'eau tout en étant très stable et en donnant un effet frais, en utilisant un émulsionnant siliconé particulier avec un rapport déterminé entre la phase huileuse et l'agent émulsionnant.

L'invention a pour objet une composition comprenant, dans un milieu physiologiquement acceptable, une phase aqueuse dispersée dans une phase huileuse à l'aide d'un agent émulsionnant siliconé, caractérisée par le fait que la phase aqueuse représente au moins 75 % en poids par rapport au poids total de la composition, que le rapport pondéral phase huileuse/agent émulsionnant est égal ou supérieur à 5 et que l'agent émulsionnant est un organopolysiloxane solide élastomère réticulé comportant au moins un groupement oxyalkyléné.

On entend par milieu physiologiquement acceptable dans la composition de l'invention, un milieu non toxique et susceptible d'être appliqué sur la peau (y compris l'intérieur des paupières) ou les lèvres d'êtres humains.

En dépit de la quantité importante d'eau, la composition de l'invention est stable dans le temps. En outre, elle possède une caractéristique rhéologique spécifique qui rend son utilisation dans les domaines considérés, particulièrement intéressante. En effet, lors de l'application sur la peau, l'émulsion "casse", c'est-à-dire qu'elle se fluidifie brutalement sous l'effet du cisaillement dû au massage sur la peau, libérant ainsi la phase aqueuse et générant une très grande sensation de fraîcheur.

La composition obtenue selon l'invention présente une viscosité allant de 2 Pa.s (20 poises) à 20 Pa.s (200 poises). Cette viscosité est mesurée au Rhéomat 180, c'est-à-dire avec l'appareil RM180 Rhéomat de la société METTLER, à température ambiante, c'est-à-dire généralement vers 20 - 25°C.

La composition selon l'invention comporte au moins 75 % en poids de phase aqueuse par rapport au poids total de la composition et de préférence au moins 80 % du poids total de la composition. La phase aqueuse peut constituer jusqu'à 92 % du poids total de la composition.

L'eau constitue au moins 65 % et de préférence au moins 70 % du poids total de la composition.

Par ailleurs, la phase aqueuse de l'émulsion peut contenir un ou plusieurs alcools inférieurs tels que l'éthanol, en une quantité allant de préférence jusqu'à 15 % et mieux jusqu'à 10 % du poids total de la composition, et/ou un ou plusieurs polyols tels que la glycérine et le propylène glycol, en une quantité allant par exemple jusqu'à 20 % et mieux jusqu'à 10 % du poids total de la composition.

La composition de l'invention contient, comme agent émulsionnant, un organopolysiloxane solide élastomère réticulé comportant au moins un groupement oxyalkyléné.

Par « élastomère » on entend un matériau souple, déformable ayant des propriétés viscoélastiques et notamment la consistance d'une éponge ou d'une sphère souple. Son module d'élasticité est tel que ce matériau résiste à la déformation et possède une capacité limitée à l'extension et à la contraction. Ce matériau est capable de retrouver sa forme originelle suite à un étirement. Cet élastomère est formé de chaînes polymériques de haut poids moléculaire dont la mobilité est limitée par un réseau uniforme de points de réticulation.

Les organopolysiloxanes de la composition de l'invention contiennent un ou plusieurs groupements oxyalkylénés et en particulier oxyéthylénés (OE), par exemple de 1 à 40 motifs oxyalkylénés et mieux 1 à 20 motifs oxyalkylénés, pouvant former des chaînes polyoxyalkylènes et notamment polyoxyéthylènes. Ces groupements peuvent être pendants, en bout de chaîne ou destinés à lier deux parties de la structure siliconée. Les atomes de silicium portant ces groupements sont au nombre d'environ 1 à 10.

Bien que l'invention concerne plus spécialement les organopolysiloxanes à groupement(s) oxyéthyléné(s), elle peut aussi concerner les organopolysiloxanes à groupement(s) oxypropyléné(s). Les organopolysiloxanes peuvent aussi comporter à la fois un ou plusieurs groupement(s) oxyéthyléné(s), 1 à 20 (OE) par exemple, et un ou plusieurs groupement(s) oxypropyléné(s) (OP), 0 à 20 par exemple ; ces organopolysiloxanes sont appelés aussi des organopolysiloxanes à groupement(s) alkyléthoxy-propyléné(s). De préférence, le nombre de groupements oxyéthylénés est supérieur au nombre de groupements oxypropylénés.

Les organopolysiloxanes élastomères utilisés dans la composition conforme à l'invention sont partiellement ou totalement réticulés et de structure tridimensionnelle. Inclus dans une phase huileuse, ils se transforment, selon le taux de phase huileuse utilisé, d'un produit d'aspect spongieux lorsqu'ils sont utilisés en présence de faibles teneurs en phase huileuse en un gel homogène, en présence de quantités de phase huileuse plus élevées. La gélification de la phase huileuse par ces élastomères peut être totale ou partielle.

Ces organopolysiloxanes élastomères peuvent se présenter sous forme de poudre, les particules constituant cette poudre ayant une taille allant généralement de 0,1 à 500 µm et mieux de 3 à 200 µm et pouvant être sphériques, plates ou amorphes avec, de préférence, une forme sphérique. Ils peuvent aussi se présenter sous forme de gel contenant l'organopolysiloxane élastomère dispersé dans une phase huileuse. Cette phase huileuse, appelée encore phase grasse liquide, peut comprendre tout corps non aqueux ou mélange de corps non aqueux, liquide à température ambiante (environ 25°C).

Les orgànopolysiloxanes élastomères pouvant être utilisés comme émulsionnant dans la composition de l'invention peuvent être choisis parmi les polymères réticulés obtenus par réaction d'addition et de réticulation en milieu non aqueux, en présence d'un catalyseur du type platine, d'au moins :
- (a) un premier organopolysiloxane (i) ayant au moins deux groupes vinyliques en position α-ω de la chaîne siliconée ; et
- (b) un second organopolysiloxane (ii) ayant au moins un atome d'hydrogène lié à un atome de silicium par molécule et au moins un groupement oxyalkyléné notamment oxyéthyléné.

En particulier, l'organopolysiloxane (i) est choisi parmi les polydiméthylsiloxanes (PDMS) et est plus spécifiquement un α-ω-diméthylvinyl polydiméthylsiloxane. L'organopolysiloxane (ii) est choisi notamment parmi les polydiméthylsiloxanes comportant un ou plusieurs atome(s) d'hydrogène lié chacun à un atome de silicium, et un ou plusieurs groupements oxyéthylénés et éventuellement un ou plusieurs groupements oxypropylénés, liés à un atome de silicium via un radical alkylène ayant de 1 à 22 atomes de carbone.

Eventuellement les chaînes silicones des premiers et seconds organopolysiloxanes (i) et (ii) comportent des chaînes pendantes alkyle en C₁ à C₆ et/ou des chaînes aryle.

Comme indiqué ci-dessus, les organopolysiloxanes élastomères utilisables dans la composition selon l'invention se présentent avantageusement dans une phase huileuse avec laquelle ils constituent un gel anhydre. Ce gel peut être notamment obtenu comme suit :
- (a) mélange du premier organopolysiloxane (i) et du second organopolysiloxane (ii) ;
- (b) ajout d'une phase huileuse au mélange de l'étape (a) ; et
- (c) polymérisation du premier organopolysiloxane (i) et du second organopolysiloxane (ii) en phase huileuse en présence d'un catalyseur de platine.

La phase huileuse utilisée lors de la fabrication du gel anhydre contient une ou plusieurs huiles liquides à températures ambiante (25°C) choisies parmi les huiles hydrocarbonées et/ou les huiles de silicone. Avantageusement, la phase huileuse est une phase liquide siliconée, contenant une ou plusieurs huiles choisies parmi les polydiméthylsiloxanes (PDMS) à chaîne linéaire ou cyclique, liquides à température ambiante et comportant éventuellement une chaîne alkyle ou aryle pendante ou en bout de chaîne, la chaîne alkyle ayant de 1 à 6 atomes de carbone.

Les organopolysiloxanes de l'invention sont en particulier obtenus selon le mode opératoire des exemples 3, 4 et 8 du document US-A-5,412,004 et des exemples du document US-A-5,811,487.

Les organopolysiloxanes de la composition de l'invention sont par exemple celui commercialisé sous la référence KSG 21 par la société Shin Etsu ou le produit de l'exemple 3 (exemple de synthèse) du brevet US-A-5,412,004.

Le KSG 21 se présente sous forme d'un gel et comprend 28 % d'organopolysiloxane et 72 % d'huile de silicone (PDMS) ayant une viscosité de 6 cSt.

Le produit de l'exemple 3 (exemple de synthèse) du document US-A-5,412,004 se présente sous la forme d'un gel pâteux contenant environ 32-33 % en poids d'organopolysiloxane réticulé à groupement(s) oxyéthyléné(s) et environ 67-68 % de PDMS 6 cSt. L'organopolysiloxane contient environ 18 % d'oxyde d'éthylène en poids par rapport au poids total du polymère. Ce gel élastomère a un comportement rhéofluidifiant plastique de 2.10⁶ poises à 4.10⁶ poises et une viscosité dynamique de 45 poises pour une vitesse de cisaillement de 200 s⁻¹, mesurée avec un rhéomètre à contrainte imposée, RS 75 (Haake) à 25°C en géométrie cône/plan ; caractéristiques du cône : 20 mm de diamètre, 1° d'angle et 40 µm de gap. Cet organopolysiloxane a, en outre, un comportement viscoélastique avec un caractère élastique dominant aux faibles valeurs de la contrainte de cisaillement, défini comme suit : 800 Pa < G * ₚₗₐₜₑₐᵤ < 2 500 Pa avec δ ₚₗₐₜₑₐᵤ voisin de 10°, G* ₚₗₐₜₑₐᵤ représentant la consistance et δ ₚₗₐₜₑₐᵤ représentant l'élasticité. Il présente un point éclair d'environ 170°C à la pression atmosphérique.

La quantité d'organopolysiloxane solide élastomère réticulé comportant au moins un groupement oxyalkyléné dans la composition de l'invention va de préférence de 0,5 à 6 % en poids de matière active et mieux de 0,6 à 5 % en poids de matière active, par rapport au poids total de la composition.

Même quand la composition est exempte de tout autre agent émulsionnant, elle présente une excellente stabilité dans le temps.

Le rapport pondéral phase huileuse/agent émulsionnant est égal ou supérieur à 5 et de préférence égal ou supérieur à 8.

La phase huileuse de la composition selon l'invention peut renfermer, outre l'huile éventuellement présente en mélange avec l'organopolysiloxane élastomère réticulé, toute sorte d'huiles et de corps gras bien connus de l'homme du métier, comme par exemple les huiles d'origine végétale (jojoba, avocat, sésame, tournesol, maïs, soja, carthame, pépins de raisin), les huiles minérales (vaseline, isoparaffines), les huiles de synthèse (myristate d'isopropyle, octanoate de cétéaryle, polyisobutylène, palmitate d'éthyl-hexyle, alkyl benzoates), les huiles de silicone volatiles ou non volatiles telles que les polydiméthylsiloxanes et les silicones cycliques (cyclodiméthylsiloxanes ou cyclométhicones), et les huiles fluorées ou fluorosiliconées, ainsi que les mélanges de ces huiles.

De préférence, la phase huileuse de la composition de l'invention comprend au moins une huile de silicone volatile généralement présente en une quantité d'au moins 4 % en poids et de préférence allant de 5 à 25 % en poids par rapport au poids total de la composition. L'huile de silicone volatile peut être choisie notamment parmi les silicones cycliques telle que la pentacyclométhicone, la tétracyclométhicone, l'hexacyclométhicone et leurs mélanges. Selon un mode particulier de réalisation de l'invention, l'huile de la phase huileuse ne comprend que des huiles de silicone volatiles.

La phase huileuse peut contenir, en outre d'autres constituants gras tels que les alcools gras comme l'alcool stéarylique, l'alcool cétylique et l'alcool cétéarylique, et les acides gras.

La phase huileuse est présente dans la composition selon l'invention en une quantité allant de 8 à 20 % et de préférence de 10 à 20 % en poids par rapport au poids total de la composition.

Un autre avantage de la composition selon l'invention provient de ce qu'on peut y incorporer une grande quantité d'au moins un électrolyte ou d'un mélange d'électrolytes sans nuire à la stabilité de la composition.

Comme électrolyte, on peut citer par exemple les sels des métaux mono-, di- ou trivalents, et plus particulièrement les sels de métal alcalino-terreux tels que les sels de baryum, de calcium et de strontium ; les sels de métal alcalin tels que les sels de sodium et de potassium, les sels de magnésium, de béryllium, d'yttrium, de lanthane, de cérium, de praséodyme, de néodyme, de prométhium, de samarium, d'europium, de gadolinium, de terbium, de dysprosium, d'holmium, d'erbium, de thulium, d'ytterbium, de lutétium, de lithium, d'étain, de zinc, de manganèse, de cobalt, de nickel, de fer, de cuivre, de rubidium, d'aluminium, de silicium, de sélénium, et leurs mélanges.

Les ions constituant ces sels peuvent être choisis par exemple parmi les carbonates, les bicarbonates, les sulfates, les glycérophosphates, les borates, les chlorures, les bromures, les nitrates, les acétates, les hydroxydes, les persulfates ainsi que les sels d'α-hydroxyacides (citrates, tartrates, lactates, malates) ou d'acides de fruits, ou encore les sels d'acides aminés (aspartate, arginate, glycocholate, fumarate).

De préférence, l'électrolyte est un mélange de sels comprenant notamment des sels de calcium, de magnésium, et de sodium, et notamment un mélange comprenant au moins du chlorure de magnésium, du chlorure de potassium, du chlorure de sodium, du chlorure de calcium, du bromure de magnésium, le dit mélange correspondant à des sels de la mer morte.

La teneur en électrolyte(s), lorsque la composition en contient, va en général de 0,5 à 20 % et de préférence de 2 à 10 % en poids par rapport au poids total de la composition.

La composition selon l'invention peut constituer notamment une composition cosmétique ou dermatologique et elle trouve son application dans un grand nombre de traitements notamment cosmétiques de la peau, y compris du cuir chevelu, des cheveux, des ongles, et/ou des muqueuses, en particulier pour le soin, le nettoyage et/ou le maquillage et/ou la protection solaire de la peau et/ou des muqueuses, ainsi que pour la préparation d'une crème destinée au traitement de la peau, plus particulièrement de la peau grasse (apport de fraîcheur).

Aussi, la présente invention a pour objet l'utilisation cosmétique de la composition telle que définie ci-dessus, pour le traitement, la protection, le soin, le démaquillage et/ou le nettoyage de la peau, des lèvres et/ou des cheveux, et/ou pour le maquillage de la peau et/ou des lèvres.

La présente invention a encore pour objet un procédé de traitement cosmétique de la peau, y compris du cuir chevelu, des cheveux, et/ou des lèvres, caractérisé par le fait que l'on applique sur la peau, les cheveux et/ou les lèvres, une composition telle que définie ci-dessus.

L'invention a aussi pour objet l'utilisation de la composition telle que définie ci-dessus pour la fabrication d'une crème destinée au traitement des peaux grasses.

De façon connue, la composition de l'invention peut contenir également des adjuvants habituels dans les domaines cosmétique et/ou dermatologique, tels que les actifs, les conservateurs, les antioxydants, les agents complexants, les solvants, les parfums, les charges, les bactéricides, les absorbeurs d'odeur, les matières colorantes et encore les vésicules lipidiques. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les vésicules lipidiques.

Comme actifs, on peut citer notamment, outre les électrolytes indiqués ci-dessus, les hydratants et par exemple les hydrolysats de protéines, et les polyols tels que la glycérine, les glycols comme les polyéthylène glycols, et les dérivés de sucre ; les extraits naturels ; les oligomères procyannidoliques ; les vitamines ; l'urée ; les dépigmentants tels que l'acide kojique et l'acide caféique ; les bêta-hydroxyacides tels que l'acide salicylique et ses dérivés ; les alpha-hydroxyacides tels que l'acide lactique et l'acide glycolique ; les rétinoïdes tels que les caroténoïdes ; les filtres, et leurs mélanges.

Le ou les actifs peuvent être par exemple présents en une concentration allant de 0,01 à 20 %, de préférence de 0,1 à 5 % et mieux de 0,5 à 3 % du poids total de la composition.

Les exemples ci-après de compositions selon l'invention sont donnés à titre d'illustration et sans caractère limitatif. Les quantités y sont données en % en poids, sauf mention contraire.

### Exemple 1 : crème pour le corps

### A. Phase huileuse

- KSG 21 à 28% de matière active 6,25 %
   (soit 1,75 % de matière active)
- Pentacyclométhicone 11,25 %

### B. Phase aqueuse

- Chlorure de sodium 2,5 %
- Eau 80 %

Mode opératoire : on prépare séparément les deux phases et on introduit la phase aqueuse dans la phase huileuse sous agitation.

On obtient une crème blanche ayant une viscosité mesurée à environ 20°C au RHEOMAT 180, de 10,5 Pa.s (105 poises) au temps zéro. Cette viscosité se stabilise après 10 minutes à 7,8 Pa.s (78 poises).

Lors de l'application sur la peau, cette crème libère de l'eau et confère une grande sensation de fraîcheur.

### Exemple 2 : crème après-solaire

### A. Phase huileuse

- KSG 21 à 28% de matière active 12,5 %
   (soit 3,5 % de matière active)
- Pentacyclométhicone 5 %

### B. Phase aqueuse

- Chlorure de sodium 2,5 %
- Eau 80 %

Mode opératoire : on prépare séparément les deux phases et on introduit la phase aqueuse dans la phase huileuse sous agitation.

On obtient une crème blanche ayant une viscosité mesurée à environ 20°C au RHEOMAT 180, de 18,7 Pa.s (187 poises) au temps zéro. Cette viscosité se stabilise après 10 minutes à 14,7 Pa.s (147 poises).

Lors de l'application sur la peau, cette crème libère de l'eau et confère une grande sensation de fraîcheur.

## Revendications

1. Composition comprenant, dans un milieu physiologiquement acceptable, une phase aqueuse dispersée dans une phase huileuse à l'aide d'un agent émulsionnant siliconé, **caractérisée par le fait que** la phase aqueuse représente au moins 75 % en poids par rapport au poids total de la composition, que le rapport pondéral phase huileuse/agent émulsionnant est égal ou supérieur à 5 et que l'agent émulsionnant est un organopolysiloxane solide élastomère réticulé comportant au moins un groupement oxyalkyléné.

2. Composition selon la revendication 1, **caractérisée en ce qu'**elle a une viscosité mesurée au viscosimètre RHEOMAT 180 à un taux de cisaillement de 200 s⁻¹ et à 25 °C, allant de 2 Pa.s à 20 Pa.s.

3. Composition selon la revendication 1 ou 2, **caractérisée en ce qu'**elle comporte au moins 65 % d'eau par rapport au poids total de la composition.

4. Composition selon l'une quelconque des revendications précédentes, **caractérisée en ce que** l'organopolysiloxane élastomère comporte au moins un groupement d'oxyéthylène.

5. Composition selon l'une quelconque des revendications précédentes, **caractérisée en ce que** l'organopolysiloxane élastomère est obtenu par réaction d'addition et de réticulation en milieu non aqueux, en présence d'un catalyseur, d'au moins : .
- un premier organopolysiloxane (i) ayant deux groupements vinyliques en position α-ω de la chaîne siliconée par molécule ; et
- un second organopolysiloxane (ii) ayant au moins un atome d'hydrogène lié à un atome de silicium par molécule et au moins un groupement oxyalkyléné.

6. Composition selon la revendication précédente, **caractérisée en ce que** le premier organopolysiloxane (i) est choisi parmi les polydiméthylsiloxanes.

7. Composition selon la revendication 5 ou 6, **caractérisée en ce que** le premier organopolysiloxane (i) est un α-ω-diméthylvinyl polydiméthylsiloxane.

8. Composition selon l'une des revendications 5 à 7, **caractérisée en ce que** le second organopolysiloxane (ii) est choisi parmi les polydiméthylsiloxanes ayant un ou plusieurs atomes d'hydrogène et un ou plusieurs groupements oxyalkylénés liés à un atome de silicium via un radical alkylène ayant de 1 à 22 atomes de carbone.

9. Composition selon l'une quelconque des revendications 5 à 8, **caractérisée en ce que** l'organopolysiloxane élastomère est sous forme d'un gel obtenu selon les étapes suivantes :
- (a) mélange du premier et second organopolysiloxanes (i) et (ii) ;
- (b) ajout d'une phase huileuse au mélange de l'étape (a) ;
- (c) polymérisation du premier et second organopolysiloxanes (i) et (ii) en phase huileuse en présence d'un catalyseur de platine.

10. Composition selon l'une des revendications précédentes, **caractérisée en ce que** l'organopolysiloxane élastomère représente de 0,5 à 6 % en poids de matière active par rapport au poids total de la composition.

11. Composition selon l'une quelconque des revendications précédentes, **caractérisée par le fait que** la phase huileuse est présente en une quantité allant de 8 à 20 % en poids par rapport au poids total de la composition.

12. Composition selon l'une quelconque des revendications précédentes, **caractérisée par le fait que** le rapport pondéral phase huileuse/agent émulsionnant est égal ou supérieur à 8.

13. Composition selon l'une quelconque des revendications précédentes, **caractérisée par le fait que** la phase huileuse contient au moins une huile de silicone volatile.

14. Composition selon l'une quelconque des revendications précédentes, **caractérisée par le fait qu'**elle contient au moins un électrolyte.

15. Composition selon la revendication précédente, **caractérisée par le fait que** l'électrolyte est présent en une quantité allant de 0,5 à 20 % du poids total de la composition.

16. Composition selon l'une quelconque des revendications précédentes, **caractérisée par le fait qu'**elle contient au moins un actif choisi parmi les hydratants, les extraits naturels, les oligomères procyannidoliques, les vitamines, l'urée, les dépigmentants, les bêta-hydroxyacides, les alpha-hydroxyacides, les rétinoïdes, les filtres, et leurs mélanges.

17. Composition selon l'une quelconque des revendications précédentes, **caractérisée en ce qu'**elle constitue une composition cosmétique.

18. Utilisation cosmétique de la composition selon l'une quelconque des revendications 1 à 17, pour le traitement, la protection, le soin, le démaquillage et/ou le nettoyage de la peau, des lèvres et/ou des cheveux, et/ou pour le maquillage de la peau et/ou des lèvres.

19. Procédé de traitement cosmétique de la peau, y compris du cuir chevelu, des cheveux, et/ou des lèvres, **caractérisé par le fait que** l'on applique sur la peau, les cheveux et/ou les lèvres, une composition selon l'une quelconque des revendications 1 à 17.

20. Utilisation de la composition selon l'une quelconque des revendications 1 à 17 pour la fabrication d'une crème destinée au traitement des peaux grasses.

## Patentansprüche

1. Zusammensetzung, die in einem physiologisch akzeptablen Medium eine mit Hilfe eines siliconierten Emulgators in einer Ölphase dispergierte wässrige Phase aufweist, **dadurch gekennzeichnet, dass** die wässrige Phase, bezogen auf das Gesamtgewicht der Zusammensetzung, mindestens 75 Gew.-% ausmacht, das Gewichtsverhältnis Ölphase/Emulgator mindestens 5 beträgt und der Emulgator ein vernetztes, festes, elastomeres Organopolysiloxan ist, das mindestens eine alkoxylierte Gruppe enthält.

2. Zusammensetzung nach Anspruch 1, **dadurch gekennzeichnet, dass** sie eine mit einem Viscosimeter RHEOMAT 180 bei einer Scherbeanspruchung von 200 s⁻¹ bei 25° C gemessene Viskosität von 2 bis 20 Pa·s aufweist.

3. Zusammensetzung nach Anspruch 1 oder 2, **dadurch gekennzeichnet, dass** sie mindestens 65 % Wasser, bezogen auf das Gesamtgewicht der Zusammensetzung, enthält.

4. Zusammensetzung nach einem der vorhergehenden Ansprüche, **dadurch gekennzeichnet, dass** das elastomere Polyorganosiloxan mindestens eine ethoxylierte Gruppe enthält.

5. Zusammensetzung nach einem der vorhergehenden Ansprüche, **dadurch gekennzeichnet, dass** das elastomere Polyorganosiloxan in einem nicht wässrigen Medium in Gegenwart eines Katalysators hergestellt wird durch Addition und Vernetzung von zumindest den folgenden Verbindungen:
- einem ersten Organopolysiloxan (i), das mindestens zwei Vinylgruppen in α-ω-Stellung der Siliconkette pro Molekül aufweist; und
- einem zweiten Organopolysiloxan (ii), das pro Molekül mindestens ein Wasserstoffatom, das an ein Siliciumatom gebunden ist, und mindestens eine alkoxylierte Gruppe aufweist.

6. Zusammensetzung nach dem vorhergehenden Anspruch, **dadurch gekennzeichnet, dass** das erste Organopolysiloxan (i) unter den Polydimethylsiloxanen ausgewählt ist.

7. Zusammensetzung nach einem der Ansprüche 5 oder 6, **dadurch gekennzeichnet, dass** das erste Organopolysiloxan (i) ein α-ω-Dimethylvinylpolydimethylsiloxan ist.

8. Zusammensetzung nach einem der Ansprüche 5 bis 7, **dadurch gekennzeichnet, dass** das zweite Organopolysiloxan (ii) unter den Polydimethylsiloxanen ausgewählt ist, die ein oder mehrere Wasserstoffatome und eine oder mehrere Oxyalkylengruppen aufweisen, die über eine Alkylengruppe mit 1 bis 22 Kohlenstoffatomen an ein Siliciumatom gebunden sind.

9. Zusammensetzung nach einem der Ansprüche 5 bis 8, **dadurch gekennzeichnet, dass** das elastomere Polyorganosiloxan in Form eines Gels vorliegt, das gemäß den folgenden Schritten hergestellt wird:
- (a) Mischen des ersten und zweiten Organopolysiloxans (i) und (ii);
- (b) Zugabe der Ölphase zu dem Gemisch aus Schritt (a); und
- (c) Polymerisation des ersten und zweiten Organopolysiloxans (i) und (ii) in der Ölphase in Gegenwart eines Platinkatalysators.

10. Zusammensetzung nach einem der vorhergehenden Ansprüche, **dadurch gekennzeichnet, dass** das elastomere Polyorganosiloxan in einer Wirkstoffmenge von 0,5 bis 6 Gew.-%, bezogen auf das Gesamtgewicht der Zusammensetzung, vorliegt.

11. Zusammensetzung nach einem der vorhergehenden Ansprüche, **dadurch gekennzeichnet, dass** die Ölphase in einer Menge von 8 bis 20 Gew.-%, bezogen auf das Gesamtgewicht der Zusammensetzung, vorliegt.

12. Zusammensetzung nach einem der vorhergehenden Ansprüche, **dadurch gekennzeichnet, dass** das Gewichtsverhältnis Ölphase/Emulgator mindestens 8 beträgt.

13. Zusammensetzung nach einem der vorhergehenden Ansprüche, **dadurch gekennzeichnet, dass** die Ölphase mindestens ein flüchtiges Siliconöl enthält.

14. Zusammensetzung nach einem der vorhergehenden Ansprüche, **dadurch gekennzeichnet, dass** sie mindestens einen Elektrolyten enthält.

15. Zusammensetzung nach dem vorhergehenden Anspruch, **dadurch gekennzeichnet, dass** der Elektrolyt in einer Menge von 0,5 bis 20 % des Gesamtgewichts der Zusammensetzung enthalten ist.

16. Zusammensetzung nach einem der vorhergehenden Ansprüche, **dadurch gekennzeichnet, dass** sie mindestens einen Wirkstoff enthält, der unter den Hydratisierungsmitteln, natürlichen Extrakten, oligomeren Procyanidinen, Vitaminen, Harnstoff, Depigmentierungsmitteln, β-Hydroxysäuren, α-Hydroxysäuren, Retinoiden, Filtern und deren Gemischen ausgewählt ist.

17. Zusammensetzung nach einem der vorhergehenden Ansprüche, **dadurch gekennzeichnet, dass** sie eine kosmetische Zusammensetzung ist.

18. Kosmetische Verwendung der Zusammensetzung nach einem der Ansprüche 1 bis 17 zur Behandlung, zum Schutz, zur Pflege, zum Abschminken und/oder zur Reinigung der Haut, der Lippen und/oder der Haare und/oder zum Schminken der Haut und/oder der Lippen.

19. Verfahren zur kosmetischen Behandlung der Haut einschließlich der Kopfhaut, der Haare und/oder der Lippen, **dadurch gekennzeichnet, dass** auf die Haut, die Haare und/oder die Lippen eine Zusammensetzung nach einem der Ansprüche 1 bis 17 aufgetragen wird.

20. Verwendung der Zusammensetzung nach einem der Ansprüche 1 bis 17 zur Herstellung einer Creme, die zur Behandlung von fettiger Haut vorgesehen ist.

## Claims

1. Composition comprising, in a physiologically acceptable medium, an aqueous phase dispersed in an oily phase using a silicone emulsifying agent, **characterized in that** the aqueous phase represents at least 75% by weight with respect to the total weight of the composition, **in that** the oily phase/emulsifying agent ratio by weight is equal to or greater than 5 and **in that** the emulsifying agent is a crosslinked elastomeric solid organopolysiloxane comprising at least one oxyalkylenated group.

2. Composition according to Claim 1, **characterized in that** it has a viscosity, measured with a Rheomat 180 viscometer at a shear rate of 200 s⁻¹ and at 25°C, ranging from 2 Pa·s to 20 Pa·s.

3. Composition according to Claim 1 or 2, **characterized in that** it comprises at least 65% of water with respect to the total weight of the composition.

4. Composition according to any one of the preceding claims, **characterized in that** the elastomeric organopolysiloxane comprises at least one oxyethylene group.

5. Composition according to any one of the preceding claims, **characterized in that** the elastomeric organopolysiloxane is obtained by an addition and crosslinking reaction in a non-aqueous medium, in the presence of a catalyst, of at least:
- one first organopolysiloxane (i) having two vinyl groups in the α,ω-position of the silicone chain per molecule; and
- one second organopolysiloxane (ii) having at least one hydrogen atom bonded to a silicon atom per molecule and at least one oxyalkylenated group.

6. Composition according to the preceding claim, **characterized in that** the first organopolysiloxane (i) is chosen from polydimethylsiloxanes.

7. Composition according to Claim 5 or 6, **characterized in that** the first organopolysiloxane (i) is an α,ω-dimethylvinylpolydimethylsiloxane.

8. Composition according to one of Claims 5 to 7, **characterized in that** the second organopolysiloxane (ii) is chosen from polydimethylsiloxanes having one or more hydrogen atoms and one or more oxyalkylenated groups bonded to a silicon atom via an alkylene radical having from 1 to 22 carbon atoms.

9. Composition according to any one of the claims 5 to 8, **characterized in that** the elastomeric organopolysiloxane is in the form of a gel obtained according to the following stages:
- (a) mixing the first and second organopolysiloxanes (i) and (ii);
- (b) adding an oily phase to the mixture of the stage (a);
- (c) polymerizing the first and second organopolysiloxanes (i) and (ii) in the oily phase in the presence of a platinum catalyst.

10. Composition according to one of the preceding claims, **characterized in that** the elastomeric organopolysiloxane represents from 0.5 to 6% by weight of active material with respect to the total weight of the composition.

11. Composition according to any one of the preceding claims, **characterized in that** the oily phase is present in an amount ranging from 8 to 20% by weight with respect to the total weight of the composition.

12. Composition according to any one of the preceding claims, **characterized in that** the oily phase/emulsifying agent ratio by weight is equal to or greater than 8.

13. Composition according to any one of the preceding claims, **characterized in that** the oily phase comprises at least one volatile silicone oil.

14. Composition according to any one of the preceding claims, **characterized in that** it comprises at least one electrolyte.

15. Composition according to the preceding claim, **characterized in that** the electrolyte is present in an amount ranging from 0.5 to 20% of the total weight of the composition.

16. Composition according to any one of the preceding claims, **characterized in that** it comprises at least one active principle chosen from moisturizing agents, natural extracts, procyanidol oligomers, vitamins, urea, depigmenting agents, β-hydroxy acids, α-hydroxy acids, retinoids, screening agents and their mixtures.

17. Composition according to any one of the preceding claims, **characterized in that** it constitutes a cosmetic composition.

18. Cosmetic use of the composition according to any one of Claims 1 to 17 for treating, protecting, caring for, removing make-up from and/or cleansing the skin, lips and/or hair and/or for making up the skin and/or lips.

19. Process for the cosmetic treatment of the skin, including the scalp, hair and/or lips, **characterized in that** a composition according to any one of Claims 1 to 17 is applied to the skin, hair and/or lips.

20. Use of the composition according to any one of Claims 1 to 17 in the manufacture of a cream intended for the treatment of greasy skin.
